# EUROPEAN PATENT SPECIFICATION

(11) **EP 0 433 463 B1**
(45) Date of publication and mention of the grant of the patent: **27.12.1995**
(21) Application number: 90909401.3
(22) Date of filing: 26.06.1990
(51) Int. Cl.: C12M 3/00, C12M 1/08

(54) **ROTARY CULTURE DEVICE**
ROTIERENDE ZUCHTANORDNUNG
DISPOSITIF ROTATIF DE CULTURE

(30) Priority: 26.06.1989 JP 160621/89; 25.06.1990 JP 166125/90
(43) Date of publication of application: 26.06.1991
(73) Proprietor: MEIJI MILK PRODUCTS COMPANY LIMITED, Tokyo 104 (JP)
(72) Inventor: MATSUMOTO, Toshiharu, Odawara-shi, Kanagawa 250 (JP); SAWAI, Yasuko, Odawara-shi, Kanagawa 250 (JP); SUZUKI, Jun, Odawara-shi, Kanagawa 250 (JP); FUZIMORI, Tatao, Meiji Milk Products Co.Ltd., Chuo-ku, Tokyo 104 (JP)
(74) Representative: Cresswell, Thomas Anthony
(86) International application number: PCT/JP90/00831
(87) International publication number: WO 91/00339

(56) References cited:
- EP-A- 205 790
- EP-A- 416 821
- CH-A- 525 959
- DE-A- 3 818 776
- FR-A- 2 264 869
- JP-A- 6 125 476
- JP-A-62 122 586

## Description

This invention is concerned with culture equipment for mass culturing of bio-organisms including animal cells, human cells, higher plant cells and cells of micro-organisms such as algae, and to maintain the cultured cells at a high density for a long period of time. In addition, this invention is concerned with devices for cell culture and cell maintenance which can be applied to culture equipment for the production of secreted substances and of cellular substances, and to bioreactors for metabolism and extracorporeal artificial organs.

The production of substances from animal cells including human cells is of great industrial utility, substances with medicinal uses and reagents such as viral antigenic proteins from virus-infected cell, interferons, cytokines, growth factors, monoclonal antibodies, and tissue plasminogen activator.

In addition, mass cells themselves, such as cultured skin keratinocytes, endothelial cells, liver cells and pancreatic Langerhans cells are of great value, since they have successfully been applied in therapy in such techniques as skin grafting, hybrid-type artificial blood vessels, artificial organs and experimental transplantation. A particular example of experimental success is the application of a mass culture of liver cells in the replacement of liver functions; a mass culture of liver cells in a vessel, which is connected to a living body through artificial blood vessels, works as an extracorporeal liver, and is expected to overcome acute liver disfunction.

Technological needs therefore exist for the methods and devices to obtain a mass culture of such bio-organisms as animal cells including human cells, as well as to maintain cultured cells for a long time in a high cell density.

Related art exemplifying the methods of animal cell culture with the purpose of substance production is described below.

### (1) Stationary monolayer culture method.

In this method, cells are cultivated whilst they adhere, either actively or passively, to the surface of a flat substratum, such as glass and plastics. Plastic culture bottles have widely been used. The use of culture vessels, each with a large surface of multi-layered plastic plates settled in a cuboid module, in mass cell culture is described in Weiss, R.S. & Schleicher, J.B. Biotech. Bioeng. 10:601-616 (1968).

This type of culture vessel is generally used in a batch culture method, i.e., a method in which cells inoculated in a vessel are fed with culture medium for a period of time, and then the culture is terminated yielding cells and spent medium. Although being the most basic method of mass cell culture for adherent cells, this method has drawbacks that the number of cells cultivated is relatively small per volume of culture vessel, that man power for handling culture vessels and feeding cells is large, and that a culture vessel is costly, if it is disposable.

### (2) Movable monolayer culture method.

In this method, a culture vessel is moved, or rotated while cells grow in a monolayer. This method partially solves problems associated with the stationary monolayer culture method. Using various mechanical means, feeding cells with gas and nutrient have become efficient. The area of substratum surface can be increased extensively. This method is exemplified by a technique (Roller bottle culture method) in which a cell monolayer is formed on the internal surface of a rolling cylindric culture bottle (roller bottle). A number of modifications of the roller bottle culture method have been attempted in order to increase cell density per device volume. In one example, a thin sheet of wavy plastic and a thin sheet of flat plastic are alternatively rolling in the interior of the roller bottle (Sterilin, Bulk Culture Vessel, Sterilin Ltd., Teddington, Middlesex).

The movable monolayer culture method increases the contact of cells with culture medium and in gas phase by rotating the culture device itself, or the culture substratum, and thus has no relation to the present invention, which essentially is a culture method in which the culture solution flows with the substratum fixed.

### (3) Micro-carrier culture method.

In this method, cells are attached to, or penetrated in micro-particles, which are suspended in-liquid medium. Thus, cells are cultivated while they are attached to, or in the floating micro-particles in suspension. Many kinds of micro-particle carrier (micro-carrier) have been devised for use in this technique. Particular attention has been paid to the development of micro-carriers suitable for weakly adhesive cells to attach to and for cells to penetrate in (Van Wezel, A.L., Nature, 216:64-65 (1967), Van Wezel, A.L. in Animal Cell Biotechnology, ed. by Spier, R.E. & Griffith, J.B., Academic Press, London, Vol. 1, 265-282 (1985)).

A vessel used for micro-carrier suspension provides a surface area for cell adhesion much larger than a vessel used for either stationary or movable monolayer culture. With this advantage, micro-carrier culture method has become a standard technique for mass cultivation of adherent cells. To maintain micro-carrier particles in a suspended state, impellers, rotators, or the introduction of floating bubbles are used. The disadvantages of this method are the difficulty in keeping micro-carriers in a homogeneous suspension and damage to cells. It has no relation to our invention: in the micro-carrier culture method, the substratum itself is floated and agitated, in the present invention, although the micro-carrier can be used as substratum, only liquid medium flows.

### (4) Cell culture method using hollow fibre.

In this method a device similar to artificial kidney for dialysis use is used. The device is composed of a bundle of may tubular semipermeable threads, hollow fibres, in a cylindrical case.

Cells are locked in the spaces between the hollow fibres, while liquid medium is circulated in the semipermeable hollow fibre in order to supply nutrient to cells and to make gas exchange through the tubular thread. Thereby, a cell density hear to that in vivo can be realised (Knazek, R.A., Gullino, P.M. Kohler, P.O. & Science, 178:65-66 (1972), Tyo, M.A., Bulbulian B.J., Menken B.Z., Murphy, T., J. Animal Cell Biotech. 3:357-371 Academic Press, London (1988)).

In addition, cells not adhering to the hollow fibre can be cultivated, since they can simply be stacked between bundles of fibres. Although frequently used, the hollow fibre culture method has no relation to the present invention, because in the hollow fibre culture method, the essential technique is characterised in that the compartment for cells and the compartment for circulating liquid medium are separated by the semi-permeable membrane of capillary tubular thread. Although hollow fibres can be used as substratum for the present invention, other substratum can also be used regardless of the semi-permeability of the fibres.

### (5) Immobilized cell culture method.

In this method, the supply of cells with nutrient medium, gas exchange, and the removal of waste products are performed by flowing liquid medium, whilst the cells are immobilised in or on the surface of substratum which permits cells to adhere, or medium to permeate. Since such common products are matrix glass fibre and ceramic monolith can be used as substrata, and since weakly adhesive cells can be immobilised, this method provides with a wide range of application.

Among techniques belonging to this culture method, that for a perfusion culture equipment (Opticell R culture equipment) is widely used. In Opticell R culture system, medium flows through a pump into a jacket with porous ceramic module incorporated therein, Opticore R, in which cells are immobilised. The medium flowing out of the module is further circulated through the channels of a gas exchanger, medium reservoir, monitoring devices, etc. (Lydersen, B.K., Pugh, G.G., Paris, M.S., Sharma, B.P., & Noll, L.A.P Biotechnology 3: 63-67 (1985)).

CH-A-525 959 discloses a cell culture device having a rotary impeller mounted in the base of a cylinder, wherein the cells are cultured on glass spheres packed outside the cylinder.

The present invention falls under the category of immobilized cell culture method in general. However, it has no relation to the prior art including Opticell® culture method. That is, this invention is concerned with a type of culture device and related techniques of culture method, while it is capable of incorporating all materials including porous ceramic module, glass fiber etc. after being worked into the water-permeable and cell-immobilizable form as substratum, it does not require a specific substratum.

### (6) Suspension culture method.

In this method, weakly adherent cells are cultivated in a floating state in a liquid medium. In principle, the method is identical to suspension culture of micro-organisms. When culturing animal cells, various devices have been installed to prevent damage to cells caused by shearing forces which occur during agitation of the culture medium. This method is widely used for modern mass culture. The method, however, has no relation to our invention which is in the category of immobilized cell culture.

The conventional cell culture methods mentioned above are associated with problems as described below.

### (1) Cell damage.

To maintain cells in a floating state, medhanical force is used, which inevitably causes shearing force on the cells. This shearing force gives rise to critical problems in suspension culture, or micro-carrier culture of animal cells which lack cell walls, and are easily damaged by sharing force. This damage becomes a critical factor preventing scaling up of culture volume, and in enhancing cell density.

### (2) Long-term storage of a culture.

Long-term storage of a culture is frequently difficult due to the wear and tear on the culture vessel, substratum and mechanical elements, and due to changes of culture environment along with the accumulation of cellular substances and/or dead cells.

Actual problems occuring with long term culture are as follows: In monolayer culture, cell layers peel-off. In suspension culture, cell separation apparatus and gas exchange apparatus clog with aggregates of cells. In micro-carrier culture, micro-carriers flocculate, and cells exfoliate. In perfusion culture such as hollow fiber culture and immobilized cell culture, increased loads on pumps often causes unexpected shut down.

### (3) Generality of culture methods.

Conventional culture methods are frequently applied only to restricted cell species depending on the existence, non-existence, or the strength of cell adherence. For example, micro-carrier culture methods and movable monolayer culture methods require strong adherence of cells to the substratum. On the other hand, adherent cells can not be usually cultivated in suspension culture.

### (4) Gas exchange

The efficiency of gas exchange in supplying oxygen for consumption and in removing carbon dioxide produced during cellular metabolism is a critical factor limiting the density of cultured cells.

In fact, in stationary monolayer culture method, low efficiency in aeration on the surface of substratum is one of the major factors limiting cell multiplication.

In suspension culture, introduction of the technique for injecting air bubbles into the liquid medium has increased the efficiency of gas exchange greatly. This technique, however, also causes problems when applied to animal cell cultures; at the surface of air bubbles, medium proteins tend to denature, and cells are damaged.

These problems have been partially solved by supplying gas through gas-permeable tubes arranged in culture medium. It is now possible with gas-permeable tubes to exchange gas with a very high efficiency. In a long term cell culture, however, gas-permeable tubes should he changes frequently since gas exchange efficiency decreases due to clogging of the surface of the tubes

### (5) The separation of cells from liquid culture medium and the supply of medium nutrients.

Cell separation in suspension culture method and micro-carrier culture method is carried out with natural precipitation, centrifuging or membrane separation. In a large scale cell culture, cell separation devices are introduced to remove waste medium, and to collect cells or a cell-adhered micro-carriers, utilizing the difference in density between cells, or a cell-adhered micro-carriers and culture medium.

The separation of cells from liquid culture medium is usually not a problem for stationary monolayer culture methods or a culture method with the state of cells adhered to a substratum. In constrast, with suspension cell culture method or micro-carrier culture method, the clogging and decreasing performance of separation apparatus is important. With modern systems having increasingly high cell density, where high performance- and long life separation apparatus are required,,cell separation methods are becoming very important.

### (6) Effect on purification process.

In a cell culture system in which a cell product is discharged into the culture medium, the load on the purification step varies considerably according to the kind of substances other than the product contained in the culture medium. In a culture system which causes a considerable damage to cells, for example, large amounts of proteins and DNA are discharged from damaged cells to culture medium, increasing the load on the purification step.

### (7) Reliability of culture equipment.

With the advancement in performance of cell culture equipment more and more parts are becoming needed for the construction and operation thereof. In addition to such main parts as culture vessel and substrate for cell culture, there are a number of parts, some of which are mentioned below for example: rotators, stirrers, impellers or vibrators, pumps and their driving parts, cocks, tubes, removable connectors for mechanical parts; and oxygen electrodes, pH electrodes, carbonic acid gas electrodes, pressure sensors, temperature sensors for monitoring parts.

In addition to these parts, there are gas supply apparatus, condensers and filters for exhausted gas, suppliers of constant temperature water, suppliers of desterilized liquid medium, sterilizing apparatus for systems and automated control installation to control total systems.

Complication of the system is inevitable. Accordingly, associated problems i.e., the high cost of culture equipment, the decrease in reliability in mechanical terms, and the insecurity of long term operation, are decisive factors with the modern large scale cell culture systems.

### (8) Economy of cell culture

With the purpose of substance production by cell culture, important factors relating to economy are the reliability, the cost and the scale of culture equipments. Cell density achieved is also an important matter. For reference, the maximum density of cells achieved is as follows: In conventional suspension culture or stationary monolayer culture, it is in the order of n x 10⁶ cells/cm³; in micro-carrier culture, it is in the order of n x 10⁷ cells/cm³; in hollow fiber culture, it is close to 10⁸/cm³. An achievement of high density near 10⁸ cells/cm³ is a current target, since there is a competitive alternative choice of production system, i.e. in vivo transplantation of hybridoma cells into animals.

An object of the present invention is to provide equipment capable of cultivating animal cells including human cells, plant cells and the cells of micro-organism including algae with minimum mechanical damage.

A further object of the present invention is to provide a culture equipment durable in long and stable storage.

A still further object of the present invention is to provide culture equipment applicable to a wide variety of micro-organisms.

A still further object of the present invention is to provide culture equipment capable of performing the exchange of nutrient components, waste products and gases between micro-organisms and culture medium with ease and high efficiency.

Still another object of the present invention is to provide a culture equipment capable of obtaining the supernatant of culture with less contaminants.

Yet another object of the present invention is to provide a culture equipment capable of achieving a high density of micro-organisms per unit volume culture medium.

Further another object of the present invention is to provide a culture equipment that is reliable in long term operation.

The present invention provides a circulatory culture apparatus for cultivating cells, comprising: a generally cylindrical vessel having an open top and a closed bottom and adapted for the circulation of a culture medium therein;
a lid for enclosing said open top;
a substratum disposed within the vessel and adapted for the growth of call cultures thereon, said substratum defining at least one central passage and a plurality of additional passages radially spaced outward from the central passage;
a wall portion extending upwardly from the bottom of the vessel to the central passage to define a circulation-generating chamber communicating with said central passage, said wall portion having a plurality of openings for the passage of the culture medium therethrough;
rotator means disposed within the circulation-generating chamber for circulating the culture medium throughout the vessel, such that rotation of the rotator means draws the culture medium down the central passage into said chamber wherein centrifugal force is imparted to the medium by the rotator means and the medium flows out said openings and up through the radially spaced passages in the substratum; and
a gas phase space defined between said lid and said substratum, said gas phase space being adapted for gas exchange between the culture medium and gas within said gas phase space.

The culture vessel has a removable lid on its top; the substratum is settled on the given position within the culture vessel with appropriate support and provides space for multiplication of micro-organism in an adherent or immobilised condition; the circulation-generating room or chamber houses mechanical means to circulate culture medium around the substratum; a circulation guiding-cyliner may be set to cover the outside of the aforementioned substratum if necessary; an air stream-guiding plate may be provided over the top of the substrate
Other optional features of the present invention are internal pressure maintaining apparatus, drying apparatus for exhaust gas filter, culture medium supplying apparatus, driving gear for rotating means, gas supply apparatus, apparatus for supplying constant temperature water, various monitoring and sensoring apparatus and automated system control installation.

A culture vessel is preferably given a shape with a high ratio of diameter against height, e.g. ca. 2:1, in order to gain high efficiency in the circulation of culture medium. The contacting surface of the bottom and the cylindric side wall of the culture vessel forms a gentle curve. The lid of culture vessel may be flat or arcuate in shape. The lid may have appartures for supplying and exhausting gases, for supplying, sampling and removing medium, and for attaching monitoring devices such as oxygen electrodes and temperature sensors. It may also have a glass viewing window. The culture vessel can be made of glass, stainless steel, and heat-resistant plastics such as polycarbonate. A glass vessel has the merit of visibility to interior.

Said substratum is cylindrically or polygonally monolithic in its outward shape, and is has a vertical hole, or a flow-down hole, passing through from the top to the bottom of the substratum. It has a number of small vertical holes or open spaces within it, and makes it water-permeable as a whole. Thus, culture medium flows up through the substantial part of said substratum vertically.

The material for the substratum in the present invention can be those having surfaces capable of adhering or immobilising micro-organisms and having certain mechanical strength.

In addition to fabrics or water permeable porous matterials such as ceramic module with honeycomb structure, materials usable include non-woven fabrics, bundles of capillary tubes, materials with layered structure or a honeycomb structure made of fibers (glass fiber, animal and plant fiber, synthetic fibers), porous matters (glass, plastics), and films. Any necessary increased capability of adhesion or immobilization of micro-organisms is obtained by a treatment of substratum surface.

A variety of substrate shapes can be designed depending on the material used for the substrate. As sated later, an important point here is to make substrate in the form that culture medium can evenly move with a sufficiently high speed within the substrate toward the top, in cooperation with circulation-generating apparatus, thus maintaining contact of fresh medium with micro-organisms propagating in the substrate. Hence, variations of form of substrate can be made on the basis of the combination with the circulatory apparatus for culture medium mentioned below.

These variations of design can be obviously included in the present invention. With a supporting device, substrate is held in a culture vessel in such a way that the top surface of substrate is held beneath an air circulation-guiding plate set in the culture vessel, that the bottom surface of substrate is held to connect the flow-down hole of the substratum with the upper opening of the circulation-generating room which contains rotating means and set on the bottom of culture vessel, and that the outer surface of the side wall of substrate is held near the inner surface of the side wall of culture vessel.

Supporting devices to held the substratum within the culture vessel as stated before can be selected depending on the material used for substratum and are not limited.

In case of woven, or unwoven textile being used for substratum, the fabric may be cut in a strip form. One end of the piece is fixed to a tubular member. Then, the fabric piece is spirally wound around the outside of the empty member with a predetermined pitch with spacers (explained later) between the textile to form a substratum fitting the inner side wall of culture vessel.

In order to maintain the winding distance between the layers of fabric textile, for instance, arms may be arranged radially with a given angle between them both at the top end and at the bottom end of the outer side wall of the empty cylindric body. The spacers are inserted into insertion holes spaced apart in the arms, and the fabric textile is wound around over the inserted spacers.

After one round of winding, the next set of spacers are inserted, and the fabric textile wound around again. When fabric textile is wound up in this way, the spaces between turns of the fabric correspond to the vertical spaces of the substratum, and the mesh of the fabric corresponds to the many small spaces of the said substratum, while the empty cylindric body corresponds to the said flowing-down hole.

A circulation-guiding cylinder, as will be mentioned later, is set in the space between the inner side wall of the culture vessel and the outer side wall of substratum. In order to stretch the rolled fabric, screws may be set to pull spacers toward the outside of the arms.

When water-permeable and porous materials such as honeycomb-like ceramic module are used for substratum. They are preferably cut so that, small spaces of the honeycomb extend vertically through the substratum, and the horizontal cross section of the substratum fits the inner wall of the vessel. It is also cut to make a cylindric vertical hole for culture medium to flow down.

Alternatively, a ceramic substratum with a concentric structure can be used, in which empty cylindric bodies made of ceramics with different diameters, are nested with the bodies of small diameters inserted in the larger.

A circulation-guiding cylinder mentioned later should be preferably set in the space between the inner side wall of culture vessel and the outer side wall of substratum. However, if there is no space, the circulation-guiding cylinder can be omitted. If a ceramic module is used as substratum, supporting devices may be needed only to fix the ceramic module on the top of the circulation-generating room within the culture vessel.

As mentioned before, the substratum is contained in, and fixed to the culture vessel with appropiate supporting devices. The circulation-generating room may be directly connected on the bottom end of the hole for flowing down of culture medium.

The circulation-generating room or chamber is positioned directly beneath the bottom end of the hole for flowing down of culture medium, and is attached to the bottom surface of the culture vessel so as to accommodate circulation-generating devices.

Thus, the circulation-generating room should have a form appropriate to high speed rotation of liquid in the room.

Possible forms are, for example, conical trapezoid with circular transverse section, inverted bowl or inverted funnel.

The circulation-generating room has a plurality of holes on its side wall permitting out-flow of culture medium. The circulation-generating room can be made of such materials as stainless steel, and heat-resistant plastics including polycarbonate.

A rotating device is accommodated in the circulation-generating room so that horizontal circulation of liquid is generated in the circulation-generating room. The device can be rotated directed by way of a driving device set outside of culture vessel, or indirectly with a magnetic stirrer.

A rotating device can be of various types, such as impeller, rod type rotator, tapered rod type rotator, and discoid rotator. The insertion of a thin plate of glass or stainless steel can protect the bottom surface of culture vessel in case the rotator touches said bottom.

The circulation-guiding may be set in the space between inner side wall of culture vessel and circumference of substratum. The circulation-guiding cylinder covers side circumferential of wall substratum.

The top end of the circulation-guiding cylinder has a height sufficient to prevent the culture medium between the inner side wall of culture vessel and the circulation-guiding cylinder phasing onto the culture medium and should be large enough for its bottom to be located at least in proximity of the bottom of the substratum in the vessel. The top of the circulation-guiding cylinder has a plurality of holes on the level corresponding to the surface of culture medium.

A gas stream-guiding plate has an opening to permit gas flow, and is set in the gas phase space between the surface of the culture medium and the lid of culture vessel. The gas stream-guiding plate covers the surface of the culture medium at a certain distance. The vertical section of the plate can have a varied shape corresponding to the shape of the vertical section of said gas phase space. For a small culture vessel, the gas stream-guiding plate can be omitted.

In addition to the apparatus described above, the following apparatus and devices can optionally be installed in culture equipment of the present invention, according to the object or scale of culture: condensers to collect water from exhaust gas; jackets to keep the exhaust gas and the filter for exhaust gas warm in order to prevent condensation of water vapor; tubes and pumps to supply and to discharge culture medium; tubes and pumps to remove the occasionally generated bubbles in the culture vessel; tubes and pumps to supply micro-organisms into the culture vessel; tubes and pumps to obtain samples; oxygen electrodes; pH electrodes; gas permeable tubes set inside of culture vessel to supply oxygen gas not only through surface aeration but through membrane aeration; gas exchange units set outside of culture vessel; jackets to keep culture vessel warm if necessary and apparatus to supply warm water to the jacket; supply sources of compressed oxygen gas, carbon dioxide gash and air, solenoid valves to adjust gas amount supplied, and in addition, tubes to connect the supply sources and the valves; internal pressure sensors for culture vessel and apparatus to prevent overpressure; medium reservoirs to store new culture medium; reservoirs for spent culture medium; various tubes to connect said apparatus and devices; connectors; switches; electronic control apparatus to control totally those apparatus, devices and things.

In addition, windows can be provided to view the inside of culture vessel; if necessary, an air pump or an externally driven fan can be also set to aid the circulation of gas within the culture vessel. As an auxiliary apparatus, an apparatus for sampling cultured cells can be fitted to the present culture equipment An example is described as follows: A small piece of material, the same as that of substratum, is attached to, or fused into one end of a glass, ceramic or metal rod. The small piece is then inserted into the interior of substratum with the other end of the rod hanging on a hook fitted to culture equipment. When cells are introduced in suspension into the substratum, they also attach to the piece of material.

After an appropriate time of incubation, the pieces can be removed from the culture apparatus under an axenic condition.

The rotating device contained in the circulation-generating room is rotated by driving force given from the outside of culture vessel. When the rotation increases, the culture medium around the rotating device rotates and moves outwardly. In this way culture medium rotating quickly in the circulation-generating room passes through the holes in the side wall of the circulation-generating room, flowing evenly from the bottom surface of the substrate into the substrate at a high speed, leading to promotion of the circulation of the medium thereof
Since the top of the circulation-guiding cylinder is set higher than the level of the culture medium surface during operation of the present culture vessel, the surface level of the culture medium stays in the space between the said cylinder and the inner wall of said vessel higher than on the substratum.

Hence, only the part of culture medium passing through the upper holes of said circulation-guiding cylinder from the said space takes part in the circulation. This system is intended to avoid possible uneven conditions in the culture vessel which might otherwise be caused by stagnant culture medium in said space. Consequently, the culture medium in said space eventually flows into the substratum resulting in efficient circulation of culture medium.

When the outer side wall of substratum attaches tightly to the inner side wall of culture vessel, culture medium flown out from the circulation-generating room with high speed is evenly led to the entire surface of the bottom of the substratum.

The culture medium flowing into the narrow vertical spaces within said substratum supplies nutrients to micro-organisms immobilized through adhesion. or entangled into the substrate, while carrying useful materials as well as waste materials produced by the micro-organisms to the top surface of the culture medium.

In due course, the culture medium then flows down through a hole opened in a part of the substratum into the circulation-generating room again. It circulates in a given direction repeating the steps mentioned above. As a result, the micro-organisms attached to, or immobilized in the substratum are cultivated.

A high density cultivation of micro-organisms can be realized depending on the effective volume of the substratum to immobilize cells, and on the reduction of damages against micro-organisms due to shearing force against them.

Furthermore, since the cells are attached to, or immobilised on the flat or uneven surface of substratum material which is kept always in a constant unidirectional flow of liquid medium, the risk that materials such as cell debris, dead bodies of cells, or other cellular substances, which float out and become contained in the supernate of culture, disturb extraction of useful materials from culture supernatant is reduced.

The use of fibres or materials with a large amount of irregular surface as substratum material reduces chances of peeling off of the cells once adhered or immobilized to the substratum. These characteristics make the long term storage of culture possible. In addition, weakly adhesive cells can stay within substratum easily.

By use of the culture equipment of present invention with a large amount of running-through spaces in the substratum and with cells adhered to or immobilized in the substratum, it is possible to change the culture medium continuously, and to collect spent medium in a short time period.

The action of gas stream-guiding plate is as follows: In the present invention, gas exchange may be carried out with gas permeable tubes (such as of silicon tubes and porous teflon tubes) installed in the bottom of the culture vessel, or outside of the culture device in modules. However, often, particularly with small culture devices of the present invention, a sufficient amount of gas exchange is possible simply by surface aeration with the arrangement of gas stream-guiding plate. The plate helps circulation of gases over the surface of culture medium and the gas exchange efficiency so that no auxiliary gas exchanger is needed.

In this surface aeration system, after gas flows from a gas supply mouth in the lid of culture vessel into culture vessel, it meets the surface of culture medium and flows along with the stream of the culture medium between the gas stream-guiding plate and the surface of culture medium, exchanging gas with the culture medium.

Then the gas reaches the hole in the gas stream guiding plate, and the gas running through the hole meets the lid and is dispersed. While a part of the gas dispersed is exhausted from the exhaust mouth in the lid, the rest flows toward the circumference between the inner surface of the top of culture vessel and gas stream-guiding plate to meet the inner side surface of the culture vessel.

Then, the gas again flows toward the hole of the gas stream-guiding plate, and repeats gas exchange between the gas stream-guiding plate and the surface of culture medium. Thus, gas circulates around both sides of gas stream-guiding plate, realizing an efficient gas exchange. When gas circulation is forcibly accelerated with an air pump or a driving fan, gas exchange efficiency is further increased by the difference between the flow of surface culture medium and the flow of gas.

The mechanical reliability of the culture equipment of the present invention exceeds that of prior arts in many respects. While the basic functions of the culture equipment, i.e., the supply and discharge of culture medium, separation of cells from culture medium, gas exchange, and circulation of culture medium, can be carried out using auxilliary outer accessary apparatus, all these functions can be carried out in a unified culture vessel in the present invention.

As movable parts of the driving body of liquid transporting pump and the rotator of pump, a peristalic pump with flexible tube and a magnetic stirrer bar can be employed respectively, so that the culture system can be completely closed avoiding the possibility of contamination. Further in the present culture equipment, it is possible to avoid the use of filters in the separation of cells from culture medium and in gas exchange, which frequently lack reliability.

With the present culture equipment, high pressure loading can be avoided in the whole tubing- and pumping system for the circulation and transportation of culture medium and gas. Thus, in comparison with other culture methods using filters and auxiliary circulation devices, the surface gas exchange method and the method of medium circulation inside the vessel used in the present culture equipment have considerably higher reliability and longer life. As summarized above, the culture equipment of the present invention can provide high mechanical reliability to all parts ranging from details to whole of the culture equipment.

The culture equipment of the present invention is more economical than that of the prior art in many respects. Reliability relating to economy was mentioned before.

An estimate of cell density to be achieved by the present invention is given as follows: In case when glass fibre is used as the substrate material in the culture equipment of the present invention, the surface area is 10 cm² per 1 cm³, when the distance between layers of glass fiber material is 2 mm, and the surface of the glass fiber material is assumed to be flat.

For comparison, CELL FACTORY®, an example of multilayered stationary culture equipment, has a volume of 12 litre per set of culture equipment, and its surface area for cell attachment is 6,000 cm².In case of cultivation with a substrate of the same volume in accordance with the present invention, the surface area for cell attachment is 120,000 cm² per 12 litre, that is, equivalent to that of 20 sets of CELL FACTORY® in terms of effective surface area of cultivation.

In case of the culture equipment of this invention, the actual volume of a culture vessel is larger than its substrate volume, since the culture vessel contains accessary apparatus in addition to the substratum. On the other hand, the effective surface area for cell attachment per unit volume of substrate is expected to be larger in the present invention than as estimated in the above, since the fibre surface has been assumed to be flat in the said estimate, and the actual fibre surface is very complex.

It is evident, therefore, that the present equipment provides larger adhesion surface area per volume than estimated. In view of the fact that CELL FACTORY® is a disposable product, the maintainance cost of culture equipment of this invention is very low due to the very low price of the material of consumed parts, such as glass fibre. There is no fundamental difficulty in extending the volume of the present circulatory culture equipment to tens of thousands of cubic meters for such purposes as growing micro-algae by extending the culture system from that for axenic to that for open.

As mentioned above, the culture equipment of the present invention is superior to those of prior arts in many respects including economy. The present invention will be described in detail hereinafter with reference to the attached figures, in which:

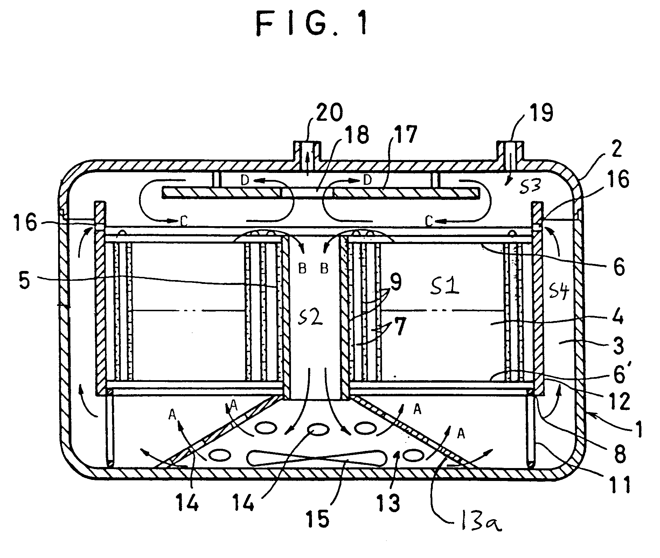

Fig. 1. is a sectional view of an example of the circulatory culture equipment of the present invention;
Figs. 2(1), (2), (3) show supporting devices for a substrate of an example;
Figs. 3(1), and (2) is a plan view of an example of a substrate supporting device with substrate installed;
Fig. 4 is a side view of an example of spacer with which substrate elements are held at a given distance; and
Fig. 5 is an oblique view of another example substrate.

Examples of culture equipment.

Hereinafter 4 examples of culture equipment, namely examples 1 - 4 will be described. In the examples 1 and 4 800 ml culture equipment), glass vessels with an internal diameter of 105 mm, internal volume of 1000 ml, and a top lid made of stainless steel, were used. In the example 2 (10 liter culture equipment), polycarbonate plastic vessels with an internal diameter of 30cm, internal volume of 10 liter, and a top lid made of stainless steel were used. In example 3 (50 liter culture vessel), stainless steel vessels, with an internal diameter of 60cm, internal volume of 50 liter, and a top lid made of stainless steel, were used. Hereinafter the 4 examples of the circulatory culture equipment of the present invention will be described in detail with reference to the drawing. All the 4 examples will be described together since all of them are essentially the same in shape and construction of the culture vessel.

The shape of the culture vessel 1 is of cylinder with a large diameter-to-height ratio, e.g. 1:2, so as to attain efficient circulation of the culture medium 3, with the circumference of the bottom surface and cylindric side wall forming a gentle curve to allow smooth circulation of the culture medium. The lid 2 has a gas inlet 19 and a gas outlet 20 as illustrated. The culture vessel 1 can have a jacket (not illustrated) around its circumference if necessary to keep culture vessel 1 warm.

In the example culture equipment 1, 2 and 3, fabric of glass fiber was used as the material for the substrate. The fabric was of plain weave of bundles of 30 single glass fibers with a thickness of 0.13mm, woven at 1mm distance, and with 75g weight/m².

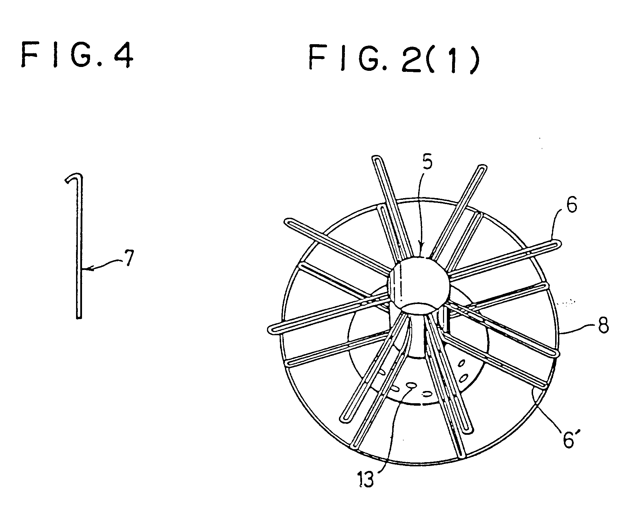

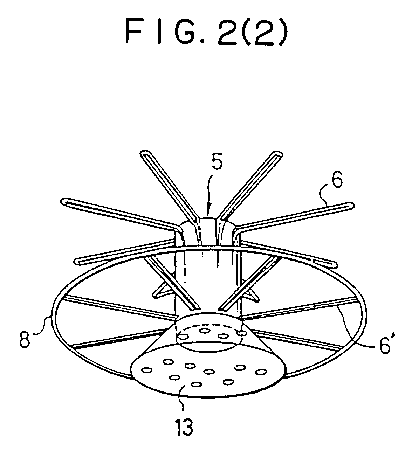

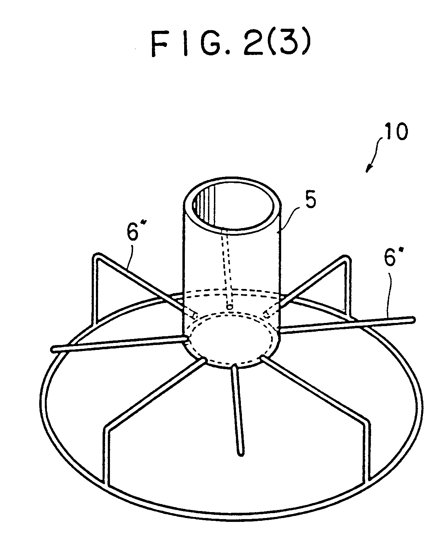

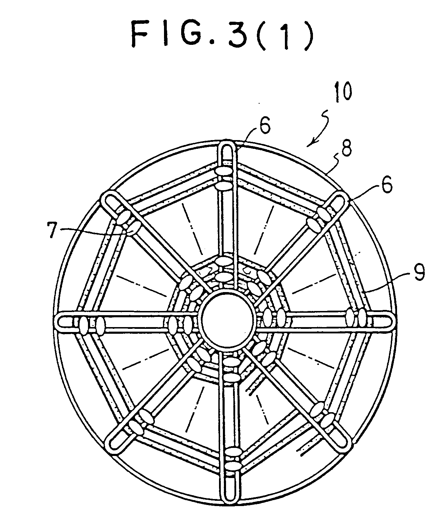

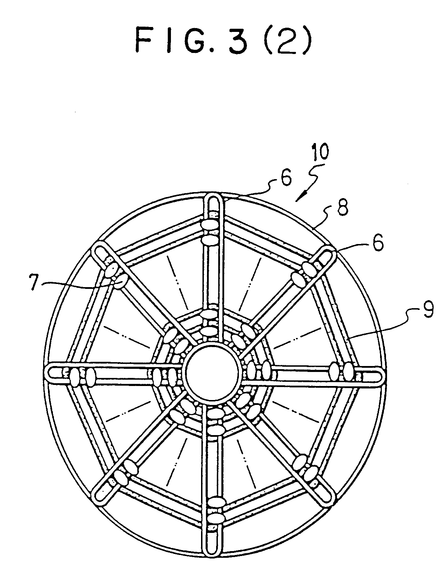

Within the culture vessel 1, the substrate is made of fabric 9 which is spirally wrapped around the support of the substratum 4 to make multilayered structure as shown in Fig. 3(1). As seen in Fig. 2(1) and (2), the support 10 of substrate is provided with arms 6 and 6' extending radially to the corresponding top and bottom ends of the circumferences of the central cylindric body 5 respectively at a predetermined spacing, and ring 8 is provided at the tip of said bottom end.

In both upper and lower arms 6, 6', there guide holes are bored into which spacer 7 can be inserted. The cylindric body 5, the upper and lower arms 6 and 6', the spacer 7, and the ring 8, are referred to as the substrate support 10. Leg 11 fitting to the bottom end of the ring 8 is installed so as to hold the substrate support 10 steady at a given position in the culture vessel 1, and to secure a given spacing (which can accommodate the conical circulation-generating room 13 and rotator 15 mentioned later) between the bottom end of the substratum support 10 (were the ring 8 is positioned) and the bottom face of the culture vessel.

As described below, in this example, fabric has been attached to the support 10 to form the substrate as shown in Fig. 3(1). Fabric 9 is cloth fabric with a width similar to the space between upper 6 and lower 6' arms and is wrapped around the cylinder body 5 to which one end of the fabric is fixed.

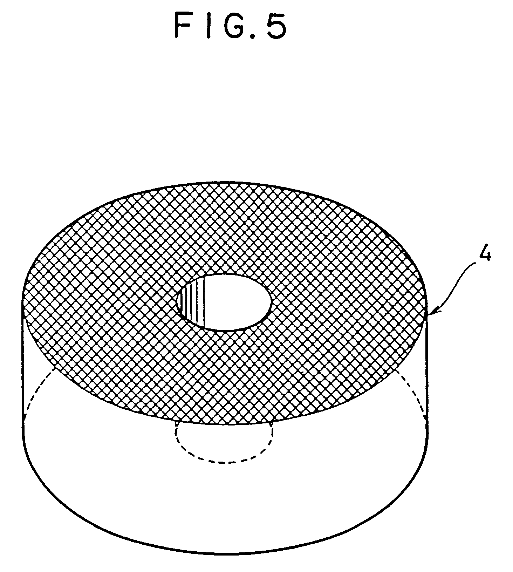

Then, in the holes of the corresponding upper and lower arms, spacers 7 with the structure shown in Fig. 4 are inserted, and placed on the fabric 9 wrapped around the cylinder body. Then the fabric 9 is wrapped around in the inserted spacers 7. These steps are repeated to make substrate 4 comprising the radially wrapped multilayered fabric 9 given a certain distance by the spacer 7 as shown in Fig. 3(1). Circulation-generating room 13, which has a conical shape, is fixed to the bottom of the cylindric body 5 of the support 10 of substrate. The top of the circulation-generating room has an opening with the same diameter as that of the cylindric body 5. The circulation-generating room 13 has several openings 14 evenly spaced on its side wall. The bottom end of the circulation-generating room touches the bottom of culture vessel 1 covering rotator 15.

The rotator 15 in the circulation-generating room 13 is driven to rotate with an outside magnetic stirrer (not illustrated). Circulation-guiding cylinder 12 is fitted to the circumference of substratum 4 to cover the substrate 4. The top end thereof protrudes from the surface of culture medium 3 and the bottom end has a length reaching near the bottom of the substrate 4. Several holes 16 are provided in the upper circumference of the circulation-guiding cylinder 12 at the level corresponding to that of the surface of culture medium 3.

In the gas phase space between culture medium 3 and the lid 2 of culture vessel, a discoid gas stream-guiding plate 17 with an opening 18 in its center is arranged to contact gas with the surface of culture medium efficiently.

In the example 4 for the preparation of culture equipment, a honeycomb-like ceramic monolith was used as the material of substrate 4 (Fig. 5). The composition of the honeycomb ceramic monolith used was MgO:Al₂O₃:SiO₂ = 2:2:5. In the monolith, honeycombs forming a regular quadrangle with a side of 2mm run vertically through the cylindrical element of 5cm height closely parallel, and a cylindrical hole 40mm in diameter is drilled on the central axis of the cylinder. A stainless-steel cylindrical body with a diameter of 38mm and height of 55mm is inserted into this inner hole of the substrate 4 (Fig. 2(3)). Supporting structure 10 was provided, which has 8 supporting rods (6") 3.2cm in length protruding horizontally and equally spaced around the circumference under the cylindrical body, and a stainless steel support 2cm in height attached to the tip of each supporting rod.

The circulation-generating room 13 was fixed to the bottom of the cylindric body 5 in the same manner as in Example 1.

Although sizes differ, the following accessary apparatus were fitted to the lid or the side surface of each culture equipment in the examples through 1 to 4:

A polarography-type oxygen electrode for each culture equipment; an interface for each electrode to send values of oxygen pressure to a computer; a solenoid valve controlled by the computer according to the value of oxygen pressure; oxygen gas supplying tube with axenic filter and a manual flow meter enabling adjustment of flow rate; a manual flow meter and a tube and axenic filter to supply air containing 5% CO₂ as the flow rate adjustable gas; a magnetic stirrer; tube and peristaltic pump to supply cell suspending solution; tube and peristaltic pump to exhaust culture medium; tube to discharge gas exhausted; warm air supplier to keep gas discharging tube and filter warm and keep them in dry condition; thermostatic bath and water pump to keep culture vessel warm by passing water into jacket.

In addition, stainless steel connectors were arranged in necessary positions to fix and remove tubes axenically. Other conditions are partly described in the examples of cultures.

The examples of this invention are constructed as described above and therefore all function as follows:
First, culture medium 3 in the bottom of culture vessel 1 rotates with the rotation of rotator 15 to generate centrifugal force. The rotating speed of the culture medium 3 is efficiently increased around the rotator 15, because the conical circulation-generating room 13 is installed so as to cover the rotator 15.

The culture medium 3 with its speed thus increased flows into substratum 4 evenly after flowing out of the holes 14 in the side wall of the conical-shaped circulation-generating room 13 as shown by arrows A.

Thus, the culture medium 3 flown into substratum rises with the substratum 4 supplying nutrients to the cells attached to the substratum 4 and discharging waste products of cells. The culture medium then reaches the top surface of the substratum 4, and overflows into cylindrical body 5 shown by arrows B. Then, it descends within the cylindrical body 5, returning into the conical circulation- generating room 13 by virtue of the action of rotator 15. These steps are repeated to enable circulation of the culture medium within substratum 4.

Since the top end of circulation-guiding cylinder 12 is higher than the level that the culture medium 3 reaches during operation, the surface of the culture medium between said cylinder 12 and the culture vessel 1 is higher than the surface of the culture medium over the substratum.

However, it is so designed that only the liquid passing through the holes 16 drilled in said cylinder 12 takes part in circulation. The concept of this partial participation at circulation is made to avoid the difficulty that various conditions within culture vessel 1 become uneven by stagnant culture medium 3 in said space.

The function of the gas stream-guiding plate 17, is described in the following. Gas flowing into culture vessel 1 through gas-supplying mouth 19 meets the surface of culture medium 3, and flows to the center of culture vessel to make gas exchange with culture medium 3.

The gas reaching the central region passes through hole 18 in the center of gas stream-guiding plate 17 meets the lid 2 and disperses around. Then, the gas flows into the direction of arrows D, and a part of the gas is discharged from mouth 20. At the edge of gas stream-guiding plate 17, the gas flowing in the direction of the arrows D meets both the flow of gas from gas supplying mouth 19 and the inner surface of the culture vessel 1, resulting in the flow into the direction of the arrows C, that is, the circulation around the gas stream-guiding plate 17.

The direction of gas flow on the surface of culture medium 3 is the same as that of the surface flow of the culture medium 3. Consequently, the circulation of gas enables efficient contact of the surface layer of the culture medium with gas, enhancing the efficiency of the gas exchange. When the circulation of gas is accelerated forcibly using a pump, gas exchange efficiency increases further.

Hereinafter examples of cell culture will be described in detail with reference to the experiments. In the examples of culture practice, the examples 1 to 4 for the preparation of culture equipments are described as type 1 culture equipment type 4 culture equipment, respectively.

The culture equipments were used after sterilization using autoclave in their constructed state.

### * Culture practice 1: Culture of various cells using type 1 culture equipment with fabric made from glass fiber.

Culture vessel 1 had a jacket with internal diameter of 105mm and depth of 105mm and a stainless steel lid. The support 10 for substratum resembled a rack made of stainless steel wire with diameter of 2mm. Eight top arms 6 and eight bottom arms 6' were arranged radially. Cylindrical body 5 made of stainless steel bad a 35mm space between the bottom of the cylindric body 5 and the bottom surface of the culture vessel 1. Substrate 4 was made of glass fiber fabric 9 of 47mm width rolled into an octagonal shape with a 2mm spacing. The substrate had 3m length and 2,820cm² surface area, in terms of the area of the fabric. The effective surface area was larger than this value due to the use of glass fiber fabric 6. For conical-shaped circulation-generating room 13 and circulation-guiding cylinder 12, polycarbonate film was used. Gas stream-guiding plate 17 was a polycarbonate disc with a diameter of 8cm and that of the hole 18 being 3cm. Rotator 15 was rotated at a speed of about 800 r.p.m. Through the lid 2 of culture vessel 1, the following five stainless steel tubes in total were distributed: a gas supply tube, a gas exhaust tube, a culture medium supply tube, a culture medium draining tube, and a tube for the supply of cell suspending solution and collection of the samples of culture medium. PH electrode and oxygen electrode were also arranged in addition.

Monolayer cultures of a line of huH-1 cultured human hepatic cancer cells (Huh, N. and Utakoji, T., Gann, 72, 178-179 (1981)) were grown in plastic dishes using DM160 culture medium supplemented with 5% bovine fetal serum (FBS) as a growth medium. The culture was treated with trypsin to obtain 1.5x10⁸ seed cells. After these cells were placed in the above mentioned culture vessel 1 filled with 800ml of culture medium for cell multiplication, the culture medium 3 was circulated through the rotation of rotator 15 to attach cell to substratum 4. During the first two days the culture medium was not renewed. During the next 20 days of total culture duration, half of the culture medium was changed on occasion to maintain glucose level above 300mg/l and lactic acid level below 500 mg/l. In the course of cultivation, air supplemented with five percent carbon dioxide gas was aerated at 5-10ml/min, and oxygen gas was added on occasion. During culturing, observation from outside of culture vessel 1 using a stereoscopic microscope was made to detect multiplication of cells attached to substratum 4. After the termination of cultivation, the fabric 9 was separated from the support 10. The separated fabric was cut into pieces to count cell nuclei using the citric acid-crystal violet staining method. Cell number was 1.05x10⁶/cm² in terms of the numbers per side of the fabric 9. The cell number per area is about two times that obtained from monolayer culture using CELL FACTORY (Nulgen Co. made). The cell number is 1.05x10⁷/cm³ in terms of cell density per volume of substratum 4.

In culture equipment type 1 with glass fibre substratum, in addition to the above mentioned human hepatic cancer cell system: a line of C127 I mouse mastocarcinoma cells (Lowry, D.R., Renal, E., Schlnick, E.M.:J.Virol. 26, 291-198 (1978) of a substratum adherent cell line; a line of KYM-1 human rhabdomyosarcoma cells adapted to grow in suspension culture with slight substratum adherence (Sekiguchi, M., Shiroko, Y., Susuki, T., Imada, M., Miyahara, M., Fujii, G.: Biomed., Pharamacother 39, 372 (1985)); and a line of mouse hybridoma cells having almost no substrate adherence (a hybridoma having mother cell from mouse myeloma of the strain X63-Ag8-6.5.3.: M. Miyahara unpublished) were cultivated. The assembly of the culture equipment, cell-seeding method and the counting method of cell numbers were conducted in accordance with those for the above mentioned huH-1 cells.

A line of C127I cells was cultivated in a growth medium consisting of Dulbecco's Modified Eagle's Medium (DME) supplemented with 5% bovine fetal serum. The cells were seeded at the density of 1.1 x 10⁴ cells/cm² in terms of one surface of glass fibre. During the course of cultivation, the exchange ratio of culture medium was gradually raised, and the whole amount was changed daily after the 10th day. Glucose consumption rate increased up to 14th day of culture to reach 60mg/h per culture equipment and levelled off thereafter. At the 23rd day of cultivation, a cell density of 5-7 x 10⁵/cm² in terms of one side of glass fibre was obtained.

A line of KYM-1 human sarcoma cell was seeded at the density of 1.6 X 10⁵ cells/cm² on the assumption that the glass fibre has two flat surfaces, and cultivated in a serum-free growth medium consisting of RPMI-1640 as the basal culture medium supplemented with trace components and 0.1% bovine serum albumin. Before seeding, the cells of this line, which show very weak adherence, had been maintained in suspension for generations. According to measurement after 17th days after the start of culture, judging from the cell floating ratio, it was estimated that ca. 45% of cells were immobilized in the substratum at that stage. The change ratio of the culture medium was gradually increased during cultivation, and after 17th day 1000ml of it was changed daily and the glucose concentration was maintained at 600mg/l. After 30 days of cultivation, a cell yield of 5.9x10⁵/cm² in terms of the surface area of fabric was achieved.

2.2x10⁸ mouse hybridoma cells, which corresponded to the density of 7.3x10⁴ cells/cm² surface area with the assumption that glass fibre is flat and two-sided, were cultivated in a growth medium consisting of RPMI-1640 culture medium supplemented with 10% FBS for 33 continuous days. During the culture duration, the 0.59 of the culture medium was changed in each day up to the eleventh day of the culture and 0.89 volume/day thereafter. Glucose concentration was maintained in the range of 1.1-1.5g/l. Judging from the glucose consumption, it can be said that this culture stabilized after the 11th day. The density of suspended cells contained in culture medium drained during this period was around 5x10⁵/ml. Cell density measured at the final stage of the culture was 6.3x10⁶/ml per volume of substratum (equivalent to 6.3x10⁵/cm² in terms of one side of glass fibre) showing that cells of about ten times the density of cells in suspension were contained in the substratum. Observation with a scanning electron microscope showed the hybridoma cells adhered to the surface of glass fibres or immobilized by being stuck therebetween, and fairly evenly distributed on the extensive area of the fibre.

The above mentioned results show that the cell culture equipment of the present invention (type 1 culture equipment) employing glass fibre as a material for substratum is applicable to immobilization, multiplication of cells, and maintenance of cultured cell of type from of strong adherence to of almost non-adherence.

### * Example 2 of culture practice: the example of culture practice using type 4 culture equipment with ceramic substratum.

The line of mouse hybridoma and the conditions of culture medium were the same as those of the examples of practice using type 1 culture equipment. The ceramic substratum used in this practical example was 400ml in volume and its surface area per unit volume is 20cm²/ml in terms of the ceramic surface regarded as flat. The surface density is twice the conversion surface density of glass fiber, which was used for type 1, 2 and 3 culture equipments. Hybridoma cells of 2.2 x 10⁸ were seeded for the present culture equipment (equivalent to 3.7 x 10⁴/cm² in terms of the ceramic surface regarded as flat)and were cultivated continuously for 34 days in a growth medium consisting of RPMI-1640 culture medium supplemented with 10% FBS. During the continuous cultivation, the renewing rate of the culture medium was gradually raised up to the tenth day of the culture. Thereafter, it was maintained at 3.5 volume/day with the concentration of glucose ranging between 1.1-1.5g/l. Judging by the consumption of glucose, this culture was almost stabilized after the eleventh day. The density of suspended cells contained in the culture medium drained in the culture period was around 5 x 10⁵/ml. No accurate measurement of cell density was conducted at the final stage of the culture in this example, but judging by the production of their monoclonal antibody, we assume that a cell density of about four times that of example using type 1 culture equipment was attained. The above results show that honeycomb-like ceramics are usable as a substrate of the culture equipment of the present invention and are able to immobilize the cells having extremely weak substratum adherence such as hybridoma cells.

### * Culture practice example 3: scale-up using type 2 and 3 culture equipments with glass made substratum.

Tests of scale up to type 2 culture equipment (10 litre scale) and type 3 culture equipment (50 litre scale) were carried out using a line of human hepatic cancer cells huH-1 and a line of human rhabdomyosarcoma cells KYM-I as follows. Fundamentally, type 2 culture equipment is composed of similar parts as the type 1 culture equipment. Culture vessel 1 is made of polycarbonate with internal diameter of 30cm and the depth of 17cm and with an outer jacket made of polycarbonate to keep it warm. The lid 2 was a disc made of stainless steel having the same seven openings as the type 1 culture equipment. The stainless steel tubes and electrodes leading to these openings are the same as those aforementioned. Substratum support 10 was made of stainless steel and was 10cm height. The central cylindrical body 5 thereof had an internal diameter of 7cm, to the top and bottom of which eight guides 8 were radially arranged. The substratum 4 was fixed to the substratum support 10 in a rolled-in state in the same way as mentioned above, with glass fibre cloth of 8.5cm x 30m rolled at 2mm spacing. Its surface area is 51,000cm² in terms of area of both surfaces regarded as flat. Conical-shaped circulation-generating room 13 was made of stainless steel. The arrangement of rotator 15 was the same as that of type 1 culture equipment described above. The gas exchange of culture medium 3 can also be done through a coiled gas exchange unit comprising 19m of gas permeable teflon tube attached to the bottom surface of the support of substratum 10.

In the test using huH-1 cells, cell culture was carried out under the same conditions of cells and multiplication culture medium as those of type 1 culture equipment previously mentioned, except that the micro-carrier culture was employed to cultivate seed cells to obtain 2.0 x 10⁹ seed cells. The seed cells obtained were seeded in culture vessel 1 filled with 10 litres of culture medium. Then, culture was continued for 28 days by stirring and circulating the culture medium at from 500 to 800 r.p.m. During the cultivation, in addition to aeration with air supplemented with 5% carbon dioxide gas, oxygen gas was supplied in the same manner as for the type 1 culture equipment. The culture medium was not renewed during the first four days. Thereafter in the present example, the total volume of culture medium was changed on occasion so as to maintain the glucose level at 400mg/l or above and the lactic acid level at 800mg/l or below. The yield of cells was 3.64 x 10⁵/cm². This value is almost equivalent to that obtained using CELL FACTORY®.

For the type 3 culture equipment (50 litre capacity), a substrate was employed, wherein a glass fiber of 10cm width and 180m length was rolled at 2mm spacing around a 12-angled stainless steel support of 60cm diameter with a cylindrical opening in the center. The diameter of culture vessel was 60cm, the depth, the internal volume of the vessel excluding lid, and the volume of substratum portion were 20cm, 50 liter and 25 liter, respectively. Whereas for supports to substratum in types 1 and 2 equipment (800ml and 10 litre capacity), a combination of arms composed of two parallel stainless steel bars and pin-shaped spacers inserted therebetween was adopted, the substratum was made by inserting the spacers, each comprising a stainless steel square bar of 5mm x 2mm thickness and 11cm length with a pair of holes drilled near their ends, into an arm comprising one stainless bar. The glass fibre was wrapped around with the advancement of the work. Auxiliary clamps for fixing the spacers toward the outer rim were attached so as to stretch the rolled fibre after completion of wrapping work. The surface area of the glass fibre was 360,000cm² in terms of one surface regarded as flat.

A line of human rhabdomyosarcoma cells (KYM-I) was used for this example. Suspension culture cells of 3 x 10¹⁰ were seeded. After cultivation for 24 hours, the number of cells remaining in suspension was counted to be 4.7 x 10⁷, by which it was estimated that the cells of 98% and more had attached to substratum. The culture was carried on using serum-free growth medium previously described, with continuous renewal of the culture medium for 16 days. During this period, glucose level was maintained in the range of 500-1000mg/l, and culture medium totalling 720 litre was supplied. Total numbers of cells were 1.87 x 10¹¹ at the completion of the culture. This value gives 5.2 x 10⁵/cm² in terms of one surface of the glass fibre to be flat, equivalent to the cell density in monolayer culture. Cells suspended in culture medium at the final stage of the culture amounted to less than 0.2% of total cell numbers.

Although not described in detail, KYM-I cells excreted useful substances in the supernatant of the culture. Cellular DNA is a contaminant causing additional burden at the separation and purification phase of useful substances, however the supernatant produced with the present culture equipment contains less than one-tenth (1/10) of the DNA contents of the supernatant obtained through the suspension culture. This fact means that the present culture equipment causes only a little damage to the cells, and that there are less chances for cellular substances to contaminate the culture supernatant.

The experiment for the human hepatic cancer cell huH-1 was successfully conducted as will be described for the long-term cultivation mentioned.

As seen in the above mentioned examples, the culture equipment according to the present invention could be easily expanded to 50 litre scale.

### Example 4: a test of long term cultivation.

Conditions on cells and growth medium were the same as those in cell culture using said 800ml culture equipment. For the seed of a line of huH-1 cells, cells were grown in monolayer in plastic dishes. The seed was treated with trypsin to obtain 3.0 x 10⁸ seed cells, which were seeded in a culture vessel 1 filled with 800 ml growth medium. Then the cells were adhered to substratum 4 through circulating culture medium 3 by rotation of rotator 15. Culture medium was not renewed for the first two days, thereafter it was changed continuously in the range from 650 ml/day to 1,950 ml/day during 19 days of growing. After the 19th day, the culture medium was changed to serum-free culture medium consisting of William E culture medium as the main component. The serum-free culture was maintained for six months. During this period, oxygen was supplied on occasion in addition to the aeration of air supplemented with 5% carbon dioxide gas at the rate of 90-95 ml/min. By stereoscopic observation through the walls of the culture vessel, we confirmed that the cells formed dense layers adhering to substratum 4 during the culture period.

During six months of cultivation, the stability in the viable state of cells was confirmed. From the cell numbers counted after the completion of the culture, the cell density of 4.4 x 10⁷/cm³ in terms of the volume of substratum 4 was attained. This equals 5.8 x 10⁶/cm² in terms of the calculation of the cell density per unit area of the fabric counting both side surfaces used for substratum in. This value is 10 times the cell density achieved in culture using CELL FACTORY®. This ratio shows that the culture equipment of the present invention is very effective as a system for substance production.

A long term culture test was carried out using human hepatic cancer huH-1 cell in type 3 culture equipment (scaled up to 50 liter). The specification of the type 3 culture equipment was basically the same as that of the aforementioned one, except for the addition of two gas exchange cylinders connecting in series each containing silicon tubular thread 2cm in diameter and 26cm in length and with bundles of silcon hollow fiber. A part of the culture medium was circulated by a peristaltic pump in a gas exchange cylinder in order to enhance gas exchange efficiency. 100% oxygen was supplied to the silicon tubular thread. This gas exchange apparatus was not used during the early stages of cultivation, but was used after the oxygen pressure of culture medium decreased due to increase of cell numbers. In this example of practice, cells of 5.9 x 10⁹ were inoculated and maintained for 209 days in culture. For the initial 26 days, DM-160 culture medium supplemented with 5% bovine fetal serum was used to make the cells grow. After this step, the culture medium was changed to serum-free medium consisting mainly of Williams E medium as described for the example for type 1 culture equipment. No troubles were observed in the culture equipment during a total of 209 days. 2.2 x 10¹¹ cells were collected. This amount of cells gives the density of 9 x 10⁶/cm³ in terms of the density per volume of substrate, and 9 x 10⁵ cells/cm² in terms of the density per one surface of the glass fiber. Although this value is lower than the density achieved by type 1 culture equipment, it is 1.8 times higher than the density by CELL FACTORY®. Though there is described not in detail in this example, the cell line used produced useful substances. In comparison with the value achieved by CELL FACTORY® , productivity of a single set of the present culture equipment is as high as that of 130 to 140 sets of CELL FACTORY with 6,000cm² culture surface area and capable of accommodating 1 liter of culture medium.

### * Measurement of gas exchange performance on liquid surface.

Permeability was measured for oxygen gas on liquid surface using type 3 culture equipment (50 litre). The glass fibre substrate was loaded into the 50 litre culture equipment, and the vessel was filled with distilled water, and then was circulated by stirrer. Immediately before the measurement, sodium sulfite was added followed by adjustment of oxygen pressure to zero, and by putting the lid over the culture equipment. The rise in oxygen pressure was measured using an oxygen electrode with air, or mixed gas of air and oxygen gas being aerated over the surface of liquid. In this measurement, circulation was not facilitated actively using a device such as surface aeration fan. However, maximum efficiency of apparent oxygen absorption was about 800mg per hour during aeration with 100% oxygen gas.

Assuming that ozygen consumption rate of normal cell is 2 »g/10⁶ cells/hour, a maximum number of cells per one culture equipment of 4 x 10¹¹, a maximum cell density of 1.6 x 10⁷/cm² per substrate volume, or of 1.6 x 10⁶/cm² per area in terms of the glass fibre surface regarded as flat, can be expected using surface aeration without particular acceleration. These values give a base to estimate maximum cell density in the present culture equipment without auxiliary apparatus for gas exchange. A higher cell density due to the relatively high rate of circulation can be expected from the small equipments type 1 and 2.

Although no details have been given in this example, axenic collection of grown cells attaching to glass fiber is possible. In case of the human hepatic cancer cells huH-1, 96% of cells were collected in a suspending solution by applying mechanical shock several times to fiber substratum from outside of culture vessel after trypsin treatment.

### EFFECT OF THE INVENTION.

The circulatory culture equipment according to this invention has many merits as follows: 1) Unlike prior culture methods such as micro-carrier culture method and suspension culture method, the culture method of present invention causes little mechanical damage to cultured cells. 2) Unlike monolayer culture method and micro-carrier culture method, the culture of present invention causes little detaching, moving, or uneven distribution of cells cultivated, so that long-term stable maintenance can be possible. 3) Regardless of the strength of the cell adhesiveness, various cells can be cultivated with the present equipment. 4) Unlike micro-carrier culture method, suspension culture method, and hollow fibre culture method, an external gas exchanger is not essential in this culture method. 5) Unlike suspension culture method and micro-carrier culture method, no apparatus for isolating cells from culture medium is needed in this culture method. 6) Unlike suspension culture method and micro-carrier culture method, there is only a little amount of contaminant cell substance in supernatant of culture solution in case of culture using this equipment. 7) Unlike hollow fibre culture method, the culture method using this equipment does not impose a large load on any pump. Unlike micro-carrier culture method and suspension culture method, the reliability in gas exchange apparatus and cell separator according to the present method is high so as to cause no anxiety.

Unlike micro-carrier culture equipment, suspension culture equipment, and hollow fiber culture equipment, the present culture equipment needs no culture medium circulation system outside of the culture equipment so that it is operated as an integrated equipment. The high reliability in biological containment thus leads to a high reliability in the total equipment. 8) The volume of the culture equipment, which frequently causes problems with the culture of adherent cells, is compact and easy to control, resulting in high reliability in the culture equipment, and in economy as the culture equipment works for a long life.

## Claims

1. A circulatory culture apparatus for cultivating cells, comprising: a generally cylindrical vessel (1) having an open top and a closed bottom and adapted for the circulation of a culture medium therein;
a lid (2) for enclosing said open top;
a substratum (4) disposed within the vessel (1) and adapted for the growth of call cultures thereon, said substratum (4) defining at least one central passage (S2) and a plurality of additional passages (S1) radially spaced outward from the central passage (S2);
a wall portion (13a) extending upwardly from the bottom of the vessel (1) to the central passage (S2) to define a circulation-generating chamber (13) communicating with said central passage, said wall portion having a plurality of openings (14) for the passage of the culture medium therethrough;
rotator means (15) disposed within the circulation-generating chamber (13) for circulating the culture medium throughout the vessel (1), such that rotation of the rotator means (15) draws the culture medium down the central passage (S2) into said chamber (13) wherein centrifugal force is imparted to the medium by the rotator means (15) and the medium flows out said openings (14) and up through the radially spaced passages (S1) in the substratum (4); and
a gas phase space (S3) defined between said lid (2) and said substratum (4), said gas phase space being adapted for gas exchange between the culture medium and gas within said gas phase space (S3).

2. An apparatus according to claim 1, wherein said lid (2) has a gas inlet (19) and a gas outlet (20), whereby gas exchange occurs between gas supplied through the inlet (19) and gases contained in the culture medium as it flows out of said radially spaced passages (S1), through the gas phase space and into the central passage (S2); and further comprising means (17) for facilitating circulation of gases in the gas phase space, including a member secured to and spaced away from said lid (2) and defining a central opening (18).

3. An apparatus according to claim 1 or 2, wherein said wall portion (13a) defines a frusto-conical circulation-generating chamber (13) such that the rotational movement of the medium within the chamber (13) is substantially unimpeded.

4. An apparatus according to claim 1, 2 or 3, further comprising:
a circulation-guiding cylinder (12) surrounding the substratum (4) and defining an annular space (S4) between said cylinder and the vessel (1) for receiving an amount of the circulating culture medium, said cylinder having a portion extending a predetermined distance beyond the substratum (4) in the direction of the vessel (1) top; and
means (16) communicating with the gas phase space (S3) for allowing a predetermined amount of culture medium from the annular space (S4) to flow into the gas phase space.

5. An apparatus according to claim 1, 2, 3 or 4, further comprising means (10) for supporting the substratum (4) including a central tubular member (5) for defining the central passage and arm members (6) radially extending from the central tubular member (5).

6. An apparatus according to claim 5, wherein the substratum (4) comprises a strip of material (9) secured to said support means (10) and wrapped around the circumference of the central tubular member (5) to form a plurality of adjacent layers of said strip of material (9), said adjacent layers being spaced apart by spacer means (7) mounted on the radial arms (6) to define the radially outward passages of the substratum (4).

7. An apparatus according to any one of claims 1 to 4, wherein said substratum (4) is composed of a honeycomb-like ceramic monolith.

## Patentansprüche

1. Zirkulations-Zuchtvorrichtung zum Kultivieren von Zellen, welche aufweist: einen im allgemeinen zylindrischen Behälter (1) mit offenem Oberteil und geschlossenem Boden, der so eingerichtet ist, daß ein Kulturmedium darin zirkulieren kann;
einen Deckel (2) zum Verschließen des offenen Oberteils;
ein innerhalb des Behälters (1) angeordnetes Substrat (4), das für das Wachstum von Zellkulturen auf dem Substrat geeignet ist, wobei das Substrat (4) mindestens einen zentralen Kanal (S2) und mehrere zusätzliche Kanäle (S1) abgrenzt, die vom zentralen Kanal (S2) radial nach außen beabstandet sind;
einen Wandteil (13a), der sich vom Boden des Behälters (1) nach oben zum zentralen Kanal (S2) erstreckt, um eine Zirkulationserzeugungskammer (13) abzugrenzen, die mit dem zentralen Kanal in Verbindung steht, wobei der Wandteil mehrere Öffnungen (14) für den Durchgang des Kulturmediums aufweist;
eine innerhalb der Zirkulationserzeugungskammer (13) angeordnete Rotationseinrichtung (15) zum Umwälzen des Kulturmediums durch den gesamten Behälter (1), derart daß das Kulturmedium durch die Rotation der Rotationseinrichtung (15) durch den zentralen Kanal (S2) nach unten in die Kammer (13) gesaugt wird, in der an dem Medium durch die Rotationseinrichtung (15) eine Zentrifugalkraft angreift, und das Medium aus den Öffnungen (14) ausströmt und durch die radial beabstandeten Kanäle (S1) nach oben in das Substrat (4) fließt; und
einen zwischen dem Deckel (2) und dem Substrat (4) abgegrenzten Gasphasenraum (S3), wobei der Gasphasenraum für einen Gasaustausch zwischen dem Kulturmedium und dem Gas innerhalb des Gasphasenraums (S3) eingerichtet ist.

2. Vorrichtung nach Anspruch 1, wobei der Deckel (2) einen Gaseinlaß (19) und einen Gasauslaß (20) aufweist, wodurch ein Gasaustausch zwischen dem durch den Einlaß (19) zugeführtem Gas und im Kulturmedium enthaltenen Gasen stattfindet, während dieses aus den radial beabstandeten Kanälen (S1) austritt, durch den Gasphasenraum und in den zentralen Kanal (S2) fließt, und die ferner eine Einrichtung (17) zur Erleichterung der Gaszirkulation im Gasphasenraum aufweist, welche ein an dem Deckel (2) befestigtes und von diesem beabstandetes Element einschließt und eine zentrale Öffnung (18) abgrenzt.

3. Vorrichtung nach Anspruch 1 oder 2, wobei der Wandteil (13a) eine kegelstumpfförmige Zirkulationserzeugungskammer (13) abgrenzt, derart daß die Rotationsbewegung des Mediums in dar Kammer (13) im wesentlichen unbehindert ist.

4. Vorrichtung nach Anspruch 1, 2 oder 3, die ferner aufweist:
einen das Substrat (4) umgebenden Zirkulationsführungszylinder (12), der einen ringförmigen Raum (S4) zwischen dem Zylinder und dem Behälter (1) abgrenzt, zur Aufnahme einer Menge des zirkulierenden Kulturmediums, wobei der Zylinder einen Teil aufweist, der um einen vorgegebenen Abstand zum Oberteil des Behälters (1) hin über das Substrat (4) hinausragt; und
eine mit dem Gasphasenraum (S3) kommunizierende Einrichtung (16), die eine vorgegebene Menge des Kulturmediums aus dem ringförmigen Raum (S4) in den Gasphasenraum fließen läßt.

5. Vorrichtung nach Anspruch 1, 2, 3 oder 4, die ferner eine Einrichtung (10) als Träger des Substrats (4) aufweist, zu der ein zentrales röhrenförmiges Element (5) zum Abgrenzen des zentralen Kanals und Armelemente (6) gehören, die in radialer Richtung von dem zentralen röhrenförmigen Element (5) ausgehen.

6. Vorrichtung nach Anspruch 5, wobei das Substrat (4) einen Materialstreifen (9) aufweist, der an der Trägereinrichtung (10) befestigt und um den äußeren Umfang des zentralen röhrenförmigen Elements (5) herumgewickelt ist, um mehrere aneinandergrenzende Schichten des Materialstreifens zu bilden, die durch an den Radialarmen (6) angebrachte Distanzstücke (7) auf Abstand gehalten werden, um die radial nach außen liegenden Kanäle des Substrats (4) abzugrenzen.

7. Vorrichtung nach einem der Ansprüche 1 bis 4, wobei das Substrat (4) aus einem wabenartigen monolithischen Keramikkörper besteht.

## Revendications

1. Appareil de culture à circulation pour cultiver des cellules, comprenant: un récipient (1) globalement cylindrique, comportant une extrémité supérieure ouverte et un fond fermé et adapté pour la circulation d'un milieu de culture se trouvant dans le récipient;
un couvercle (2) pour fermer l'extrémité supérieure ouverte;
un substrat (4) disposé dans le récipient (1) et adapté pour la croissance de cultures de cellules se trouvant sur ce substrat, ledit substrat (4) définissant au moins un passage central (S2) et une pluralité de passages supplémentaires (S1) espacés radialement vers l'extérieur depuis le passage central (S2);
une partie formant paroi (13a) s'étendant vers le haut depuis le fond du récipient (1) jusqu'au passage central (S2) pour définir une chambre (13) de génération de circulation, communiquant avec ledit passage central, ladite partie formant paroi comportant une pluralité d'ouvertures (14) pour le passage du milieu de culture à travers ces ouvertures;
un moyen (15) rotatif disposé à l'intérieur de la chambre (13) de génération de circulation, pour faire circuler le milieu de culture à travers le récipient (1), de telle sorte que la rotation du moyen rotatif (15) attire le milieu de culture vers le bas dans le passage central (S2) jusque dans ladite chambre (13) où une force centrifuge est communiquée au milieu par le moyen rotatif (15) et le milieu s'écoule hors desdites ouvertures (14) puis vers le haut à travers les passages (S1) espacés radialement dans le substrat (4); et
un espace (S3) pour phase gazeuse, défini entre ledit couvercle (2) et ledit substrat (4), ledit espace pour phase gazeuse étant adapté pour un échange de gaz entre le milieu de culture et le gaz se trouvant à l'intérieur dudit espace (S3) pour phase gazeuse.

2. Appareil selon la revendication 1, dans lequel ledit couvercle (2) comporte une entrée (19) de gaz et une sortie (20) de gaz, grâce à quoi un échange de gaz a lieu entre le gaz amené à travers l'entrée (19) et les gaz contenus dans le milieu de culture au fur et à mesure qu'il sort desdits passages (S1) espacés radialement, traverse l'espace pour phase gazeuse et pénètre dans le passage central (S2); et comprenant, en outre, un moyen (17) pour faciliter la circulation des gaz dans l'espace pour phase gazeuse, un élément étant fixé audit couvercle (2) en en étant espacé et définissant une ouverture centrale (18).

3. Appareil selon la revendication 1 ou 2, dans lequel ladite partie formant paroi (13a) définit une chambre tronconique (13) de génération de circulation, de telle sorte que le mouvement de rotation du milieu à l'intérieur de la chambre (13) est sensiblement libre.

4. Appareil selon la revendication 1, 2 ou 3, comprenant, en outre:
un cylindre (12) de guidage de circulation entourant le substrat (4) et définissant un espace annulaire (S4) entre ledit cylindre et le récipient (1) pour recevoir une certaine quantité du milieu de culture en circulation, ledit cylindre comportant une partie s'étendant sur une distance prédéterminée au-delà du substrat (4) dans la direction de l'extrémité supérieure du récipient (1); et
un moyen (16) communiquant avec l'espace (S3) pour phase gazeuse afin de permettre à une quantité prédéterminée de milieu de culture de s'écouler depuis l'espace annulaire (S4) jusque dans l'espace pour phase gazeuse.

5. Appareil selon la revendication 1, 2, 3 ou 4, comprenant, en outre, un moyen (10) pour supporter le substrat (4), comprenant un élément tubulaire central (5) pour définir le passage central et les éléments formant bras (6) s'étendant radialement depuis l'élément tubulaire central (5).

6. Appareil selon la revendication 5, dans lequel le' substrat (4) comprend une bande de matériau (9) fixé audit moyen de support (10) et enroulé autour de l'élément tubulaire central (5) pour former une pluralité de couches adjacentes de ladite bande de matériau (9), lesdites couches adjacentes étant espacées les unes des autres par des moyens d'espacement (7) montés sur les bras radiaux (6) de manière à définir les passages du substrat (4) qui s'étendent radialement vers l'extérieur.

7. Appareil selon l'une quelconque des revendications, 1 à 4, dans lequel ledit substrat (4) est composé d'un monolith céramique analogue à un nid d'abeilles.
